# FASCICULE DE BREVET EUROPEEN

(11) **EP 4 076 619 B1**
(45) Date de publication et mention de la délivrance du brevet: **08.04.2026**
(21) Numéro de dépôt: 20845421.5
(22) Date de dépôt: 15.12.2020
(51) Int. Cl.: A61M 39/08, A61M 39/20

(54) **ACCESSOIRE POUR L'IMPLANTATION D'UN DISPOSITIF MEDICAL CONFIGURE POUR OBTURER UN CONDUIT ANATOMIQUE**
ZUBEHÖR ZUM IMPLANTIEREN EINES MEDIZINPRODUKTS ZUR VERSIEGELUNG EINES ANATOMISCHEN KANALS
ACCESSORY FOR IMPLANTING A MEDICAL DEVICE CONFIGURED TO SEAL AN ANATOMICAL DUCT

(30) Priorité: 16.12.2019 FR 1914521
(43) Date de publication de la demande: 26.10.2022
(73) Titulaire: Uromems, 38320 Eybens (FR)
(72) Inventeur: MOZER, Pierre, 94300 Vincennes (FR); BEAUGERIE, Aurélien, 92130 Issy-les-Moulineaux (FR); MIR, Riaz, 38600 Fontaine (FR)
(74) Mandataire: Regimbeau
(86) Numéro de dépôt international: PCT/FR2020/052447
(87) Numéro de publication internationale: WO 2021/123605

(56) Documents cités:
- EP-A1- 3 290 683
- DE-U1- 9 419 630
- US-B1- 7 597 117

## Description

### Domaine technique

L'invention concerne un accessoire pour l'implantation d'un dispositif médical dans un corps humain ou animal configuré pour obturer un conduit anatomique.

### Etat de la technique

Il existe des dispositifs médicaux adaptés pour obturer sélectivement un conduit anatomique, tel que l'urètre, le col vésical ou l'estomac.

Pour lutter contre l'incontinence urinaire, il est connu d'implanter chez un patient un dispositif médical comprenant une manchette occlusive gonflable agencée autour de l'urètre ou du corps vésical, un réservoir de fluide et une pompe permettant de transférer du fluide entre la manchette et le réservoir afin de sélectivement gonfler ou dégonfler la manchette pour assurer une compression déterminée de l'urètre ou du col vésical.

La liaison fluidique entre ces différents composants est assurée par un ou plusieurs tubes.

La connexion du tube avec les composants est réalisée lors de l'intervention chirurgicale d'implantation.

Avant l'implantation, le dispositif médical est rempli d'un fluide biocompatible et le circuit fluidique est purgé afin d'éviter la présence de bulles d'air.

Afin de maintenir le circuit fluidique purgé et éviter de contaminer l'intérieur du tube et la partie du dispositif médical associée audit tube, les extrémités du tube sont obturées par un bouchon pendant l'implantation jusqu'à la connexion effective du tube avec les composants. La fixation de chaque bouchon doit être suffisamment forte pour résister à des forces de traction importantes exercées pendant l'implantation.

En pratique, les bouchons sont fixés au tube par des sutures.

Cependant, suturer chaque bouchon aux extrémités de chaque tube constitue une opération spécifique du chirurgien qui allonge la durée totale de l'intervention chirurgicale et peut entraîner des complications, par exemple une déconnexion du bouchon et du tube. Les documents DE9419630U1, US7597117B1 et EP3290683A1 décrivent tous des bouchons pour tube.

Un objet de l'invention est donc de faciliter et sécuriser la connexion entre le bouchon et le tube.

### Brève description de l'invention

Un but de l'invention est donc de concevoir un accessoire permettant de simplifier l'intervention chirurgicale.

A cet effet, un premier objet de l'invention concerne un accessoire pour une implantation d'un dispositif médical dans un corps humain ou animal, ledit dispositif médical comprenant une manchette occlusive adaptée pour entourer et obturer un conduit anatomique dudit corps humain ou animal, un réservoir de fluide et un tube reliant le réservoir à la manchette occlusive, ledit accessoire comprenant :
- un bouchon adapté pour obturer une extrémité du tube, et
- un collet adapté pour entourer le tube,
le bouchon et le collet présentant des portions de connexion respectives adaptées pour coopérer de manière à fixer le collet et le bouchon ensemble de sorte à obturer hermétiquement ladite extrémité du tube.

Avantageusement, on supprime ainsi la nécessité de recourir à une suture pour fixer le bouchon à l'extrémité du tube.

Dans certains modes de réalisation les portions de connexion du bouchon et du collet sont agencées de sorte à permettre un emboîtement du collet avec le bouchon.

Dans certains modes de réalisation, l'une des portions de connexion du collet ou du bouchon présente au moins une protrusion et l'autre portion de connexion du collet ou du bouchon (qui ne comprend pas ladite protrusion) présente au moins une cavité, ladite au moins une protrusion étant déformable élastiquement pour permettre l'engagement de ladite au moins une protrusion dans ladite au moins une cavité.

Dans certains modes de réalisation, le bouchon comprend en outre une tige de forme allongée de sorte à former un guide d'insertion du tube lors de l'implantation.

Par « forme allongée » on entend une forme dont la longueur est supérieure à 10 fois la dimension transversale (par exemple le diamètre dans le cas d'une section circulaire). Dans certains modes de réalisation, le bouchon comprend un embout adapté pour être inséré dans une extrémité du tube.

En accord avec l'invention, la portion de connexion du bouchon comprend une gorge annulaire entourant ledit embout configurée pour recevoir une extrémité déformable élastiquement du collet de sorte à pincer l'extrémité du tube.

Dans certains modes de réalisation, le bouchon présente au moins une zone de préhension pour une pince configurée pour connecter le collet au bouchon.

Dans certains modes de réalisation, ladite zone de préhension comprend deux méplats parallèles bordés par une collerette.

Dans certains modes de réalisation, lesdits méplats sont traversés par un orifice de passage d'une suture.

Dans certains modes de réalisation, l'accessoire comprend un capuchon adapté pour entourer le bouchon de sorte à assurer une continuité de la surface extérieure du bouchon en regard de la zone de préhension.

Dans certains modes de réalisation, ledit capuchon comprend deux demi-coques emboîtables de part et d'autre du bouchon.

Dans certains modes de réalisation, lesdites demi-coques sont articulées par une charnière.

Un autre objet concerne un kit d'implantation pour une implantation d'un dispositif médical dans un corps humain ou animal, ledit dispositif médical comprenant une manchette occlusive adaptée pour entourer et obturer un conduit anatomique dudit corps humain ou animal, un réservoir de fluide et un tube reliant le réservoir à la manchette occlusive.

Ledit kit comprend avantageusement un accessoire tel que décrit plus haut, et une pince incluant un premier mors adapté pour recevoir une portion du bouchon opposée à la portion de connexion dudit bouchon et un second mors adapté pour recevoir une portion du collet opposée à la portion de connexion dudit collet, lesdits mors étant mobiles entre une position ouverte adaptée pour l'engagement du collet et du bouchon avec chaque mors respectif et une position fermée adaptée pour connecter le collet et le bouchon par leurs portions de connexion respectives.

Un autre objet concerne un ensemble comprenant un dispositif médical et un accessoire tel que décrit ci-dessus pour l'implantation dudit dispositif médical dans un corps humain ou animal, ledit dispositif médical comprenant une manchette occlusive adaptée pour entourer et obturer un conduit anatomique dudit corps humain ou animal, un réservoir de fluide et un tube reliant le réservoir à la manchette occlusive.

La mise en oeuvre d'un tel accessoire au cours de l'implantation d'un dispositif médical dans un corps humain ou animal peut être la suivante :
- engagement du collet sur un tube, la portion de connexion du collet étant située du côté de l'extrémité à obturer du tube ;
- engagement de la portion de connexion du bouchon avec la portion de connexion du collet, de sorte à pincer l'extrémité du tube et à solidariser le bouchon vis-à-vis du collet,
- insérer le bouchon obturé par l'accessoire dans le corps humain ou animal jusqu'à un site d'implantation.

### Brève description des dessins

D'autres caractéristiques et avantages de l'invention ressortiront de la description détaillée qui va suivre, en référence aux dessins annexés, sur lesquels :
La figure 1 est une vue schématique d'un dispositif médical dont l'implantation est adaptée à la mise en oeuvre de l'accessoire ;
La figure 2 est une vue en perspective d'un bouchon selon un mode de réalisation ;
La figure 3 est une vue en perspective d'un collet selon un mode de réalisation ;
La figure 4 est une vue en perspective d'un bouchon selon un autre mode de réalisation ;
La figure 5 illustre un capuchon adapté pour assurer une continuité de la surface extérieure du bouchon en regard des zones de préhension ;
La figure 6 est une vue en perspective de la connexion du bouchon de la figure 2 et du collet de la figure 3 au moyen d'une pince.
La figure 7 est une vue en perspective de la connexion du bouchon de la figure 4 et du collet de la figure 3 au moyen d'une pince.

### Description détaillée de modes de réalisation

La présente invention trouve entre autres une application, sans pour autant y être limitée, pour un certain nombre de dispositifs médicaux, parmi lesquels par exemple des sphincters artificiels, des muscles artificiels, des stimulateurs électriques, des anneaux gastriques, des neurostimulateurs, les prothèses vasculaires ou des implants péniens. Toutefois, la présente invention peut être mise en oeuvre dans toute autre application nécessitant la formation d'un passage dans lequel on souhaite introduire une forme allongée telle qu'un tube.

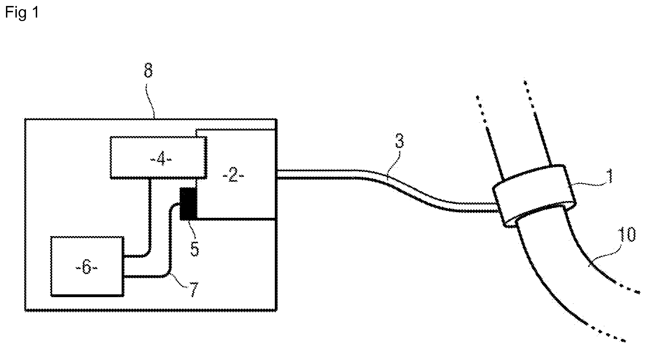

La figure 1 illustre de manière schématique et à titre non limitatif un sphincter urinaire artificiel dont l'implantation peut être facilitée par l'utilisation d'un accessoire selon l'invention. Ledit dispositif médical comprend un élément occlusif adapté pour entourer un conduit naturel 10. L'élément occlusif se présente sous la forme d'une manchette gonflable 1 susceptible d'être remplie d'une quantité variable d'un fluide, une variation de la pression de fluide à l'intérieur de la manchette faisant varier la compression exercée sur le conduit naturel 10 à occlure.

Un réservoir 2 d'un fluide biocompatible, par exemple du sérum physiologique, est agencé en liaison fluidique avec la manchette, par l'intermédiaire d'un tube 3.

L'ensemble de la manchette 1, du réservoir 2 et du tube 3 forme le circuit fluidique du dispositif médical.

Ce circuit fluidique permet de transférer une partie du fluide du réservoir 2 dans la manchette, afin d'augmenter la compression exercée sur le conduit 10, et inversement de transférer une partie du fluide de la manchette 1 vers le réservoir 2, pour diminuer la compression exercée par la manchette sur le conduit 10.

A cet effet, le système occlusif comprend en outre un dispositif d'actionnement 4 pour effectuer ce transfert de fluide et ainsi faire varier la compression exercée par la manchette sur le conduit 10.

De manière particulièrement avantageuse, le réservoir 2 présente un volume variable.

Par exemple, mais de manière non limitative, la variation de volume peut être réalisée en déplaçant une paroi du réservoir, le dispositif d'actionnement 4 comprenant un actionneur pour déplacer ladite paroi par exemple selon l'agencement décrit dans le document EP3223748B1. Ainsi, le réservoir peut comprendre une membrane roulante, un piston, un soufflet ou tout autre moyen permettant de faire varier son volume. L'homme du métier est en mesure de sélectionner parmi les actionneurs existants un actionneur adéquat en fonction de l'implémentation envisagée vis-à-vis du réservoir. On peut citer par exemple mais de manière non limitative un actionneur piézo-électrique.

Le dispositif médical comprend un capteur permettant de mesurer l'action exercée sur le réservoir. Par exemple, si l'actionnement consiste en un déplacement d'une paroi mobile du réservoir, ledit capteur peut consister en un capteur 5 de position, permettant de déterminer la position de la paroi mobile. Un calibrage permet de déterminer d'une part, la relation entre la position de la paroi mobile et la variation de volume du réservoir ; d'autre part, la relation entre la variation de volume du réservoir et la pression dans le circuit hydraulique et enfin entre la pression dans le circuit hydraulique et la compression exercée sur le conduit à occlure. Ainsi, il est possible de déterminer le déplacement à imposer à la paroi mobile pour obtenir une pression donnée de fluide dans le circuit hydraulique en vue d'obtenir une compression donnée du conduit 10 par la manchette 1.

Le système occlusif comprend en outre une unité de commande 6 adaptée pour solliciter le dispositif d'actionnement 4 de sorte à exercer une compression déterminée sur le conduit 10. La liaison 7 entre l'unité de commande 6 et le dispositif d'actionnement 4 a été représentée sous forme filaire sur la figure 1, mais il va de soi qu'elle pourrait être mise en oeuvre sans fil, selon la technologie sélectionnée par l'homme du métier. Il existe également une liaison 7 (filaire ou non) entre le capteur 5 et l'unité de commande 6.

Le réservoir 2, le dispositif d'actionnement 4, l'unité de commande 6 et le capteur 5 sont agencés dans un boîtier 8 étanche en un matériau biocompatible. Le boîtier 8 comprend une sortie fluidique qui est raccordée de manière étanche au tube 3, assurant la liaison fluidique entre le réservoir 2 et le tube 3.

En vue de l'implantation, la manchette 1 et une partie au moins du tube 3 sont fournis dans un emballage stérile, séparément du boîtier. Le boîtier 8 est également fourni dans un emballage stérile, avec une autre partie du tube 3 déjà raccordée à la sortie fluidique du boîtier. Le réservoir 2 et, le cas échéant, le tube 3, et la manchette 1 sont fournis vides du fluide biocompatible.

Dans le cas où le réservoir 2, le tube 3 et la manchette 2 sont fournis vides de fluide, une étape préalable à l'implantation consiste alors à purger le circuit fluidique de l'air contenu initialement et à le remplir du fluide biocompatible.

Pendant l'implantation, le circuit fluidique est maintenu purgé par l'accessoire qui va être décrit plus bas.

Pour implanter le dispositif médical, le praticien doit mettre en place la manchette autour du conduit anatomique à occlure, mettre en place le boîtier dans une région anatomique voisine du conduit naturel 10, et raccorder la sortie fluidique à la manchette 1 par l'intermédiaire du tube 3.

Pour faciliter ladite implantation du dispositif médical, il est proposé un accessoire formé de deux parties emboîtables, à savoir un bouchon adapté pour obturer une extrémité du tube 3, et un collet adapté pour entourer le tube 3. Le collet et le bouchon présentent des portions de connexion respectives adaptées pour coopérer de manière à fixer le collet et le bouchon ensemble de sorte à obturer hermétiquement ladite extrémité du tube 3.

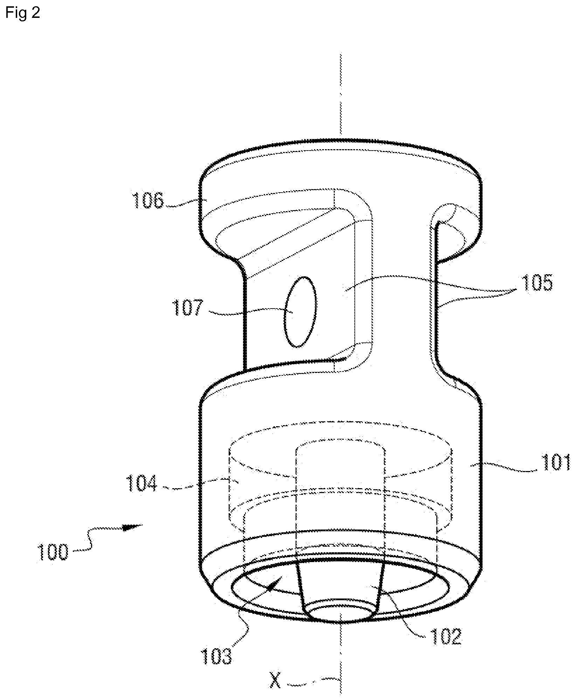

La figure 2 est une vue en perspective d'un mode de réalisation du bouchon.

Le bouchon 100 s'étend selon un axe longitudinal X.

Le bouchon 100 comprend une paroi circonférentielle 101 qui est inscrite dans un cylindre d'axe de révolution X.

Une extrémité du bouchon 100 comprend un embout 102 adapté pour être inséré dans le tube 3. La surface extérieure de la paroi circonférentielle 101 définit, avec l'embout 102, une gorge annulaire 103 adaptée pour recevoir la paroi du tube. Le diamètre de l'embout 102 est sensiblement égal au diamètre intérieur du tube 3, de préférence légèrement supérieur audit diamètre intérieur pour permettre un emmanchement serré du tube sur l'embout 102, de façon à assurer une liaison étanche entre le tube 3 et l'embout 102. L'embout 102 présente une forme pleine, de sorte à obturer le tube lorsque celui-ci est emmanché sur l'embout 102 qui pénètre ainsi dans l'intérieur du tube 3.

En regard de l'embout 102, la surface intérieure de la paroi circonférentielle 101 comprend une cavité 104 qui s'étend sur au moins une partie de cette surface intérieure.

Dans certains modes de réalisation (non illustrés), ladite cavité s'étend au travers de la paroi circonférentielle 101, formant ainsi une ouverture radiale communiquant avec la gorge annulaire. Une telle ouverture peut en particulier permettre de vérifier visuellement que le tube 3 est bien emmanché sur l'embout 102.

La gorge annulaire 103 et la cavité 104 forment une portion de connexion du bouchon sur le collet.

Du côté opposé à la portion de connexion dans la direction de l'axe longitudinal X, la paroi circonférentielle 101 est pourvue de deux zones 105 de préhension du bouchon par une pince. Lesdites zones de préhension 105 peuvent se présenter sous la forme de deux méplats formés de part et d'autre d'un plan de symétrie du bouchon passant par l'axe longitudinal X. De manière plus générale, lesdites zones de préhension peuvent être définies par une diminution localisée de l'épaisseur de la paroi circonférentielle 101. L'extrémité de la paroi circonférentielle forme une collerette 106 délimitant lesdites zones de préhension 105 et servant d'appui pour le maintien des mors d'une pince sur les zones de préhension 105. Au niveau desdites zones de préhension 105, la paroi circonférentielle peut présenter un orifice traversant 107. Ledit orifice peut permettre le passage d'une suture afin de faciliter la manipulation du bouchon lors de l'implantation.

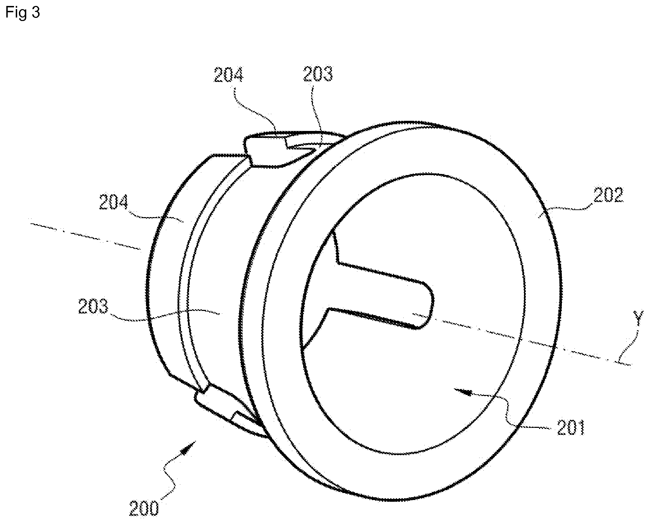

La figure 3 est une vue en perspective d'un mode de réalisation du collet.

Le collet 200 s'étend selon un axe longitudinal Y qui, dans la configuration assemblée de l'accessoire, est confondu avec l'axe longitudinal X.

Le collet 200 comprend une ouverture traversante 201 disposée dans sa partie centrale autour de l'axe longitudinal Y.

A l'une de ses extrémités, le collet 200 comprend une collerette 202 qui s'étend radialement vers l'extérieur. De manière avantageuse, ladite collerette présente un profil arrondi, non agressif pour les tissus anatomiques rencontrés au cours de l'implantation.

A l'extrémité opposée de la collerette 202 selon la direction longitudinale Y, le collet 200 comprend plusieurs pattes flexibles 203 séparées par des intervalles formant des espaces libres, orientées selon la direction longitudinale Y. Chaque patte 203 comprend une protrusion 204 s'étendant radialement vers l'extérieur. Les pattes flexibles 203 et protrusions 204 forment une portion de connexion du collet au bouchon.

En vue de l'implantation, le collet 200 est emmanché autour du tube, le sens d'emmanchement étant choisi de sorte à placer la portion de connexion du côté de l'extrémité du tube à obturer. En d'autres termes, les pattes 203 sont relativement plus proches que la collerette 202 de l'extrémité du tube à obturer.

Par ailleurs, la portion de connexion du bouchon 100 est engagée dans la portion de connexion du collet 200. Ce faisant, l'embout 102 est inséré à l'extrémité et dans l'intérieur du tube 3 et la paroi extérieure du tube 3 et les pattes 203 du collet sont reçues dans la gorge annulaire 103.

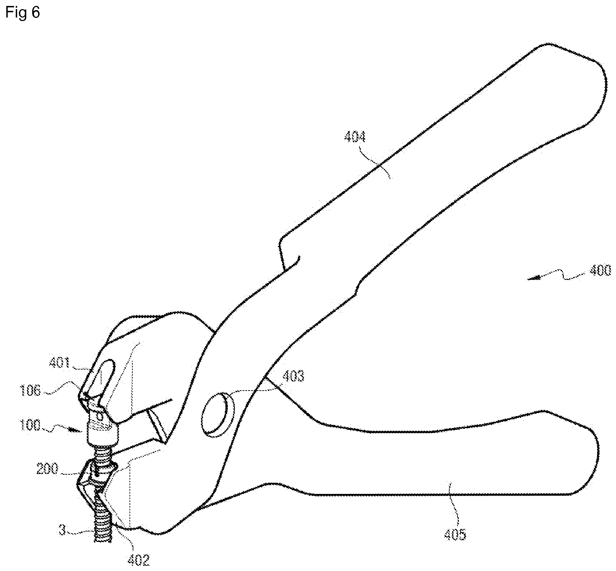

Le bouchon est rendu solidaire du collet par engagement des protrusions 204 des pattes flexibles 203 dans la cavité 104. A cet effet, le praticien utilise avantageusement une pince pour faciliter cet engagement en exerçant une pression sur le collet et le bouchon dans des direction opposées et selon un mouvement de fermeture des mors de la pince (cf. figure 6). La largeur de la gorge annulaire 103 est sensiblement égale ou légèrement inférieure à la somme des épaisseurs du tube et des pattes flexibles 203, la cavité 104 étant quant à elle dimensionnée pour pourvoir recevoir chaque protrusion 204 après déformation élastique de chaque patte 203 pour s'engager dans ladite gorge annulaire 103. De manière avantageuse, les protrusions 204 et l'embouchure de la gorge annulaire 103 présentent des chanfreins respectifs adaptés pour faciliter l'engagement du collet 200 dans le bouchon 100.

Naturellement, les protrusions et la gorge pourraient être remplacés par d'autres moyens de connexion aptes à coopérer pour procurer un emboîtement ferme. En particulier, la portion de connexion mâle (telle que les protrusions 204) peut être agencée sur le collet ou sur le bouchon, et la portion de connexion femelle (telle que la gorge et la cavité) peut être inversement agencée dans le bouchon ou dans le collet.

Une fois le bouchon 100 et le collet 200 solidaires l'un de l'autre, l'extrémité du tube est pincée entre l'embout 102 et les pattes 203, ce qui assure une étanchéité fluidique entre le tube 3 et le bouchon 100.

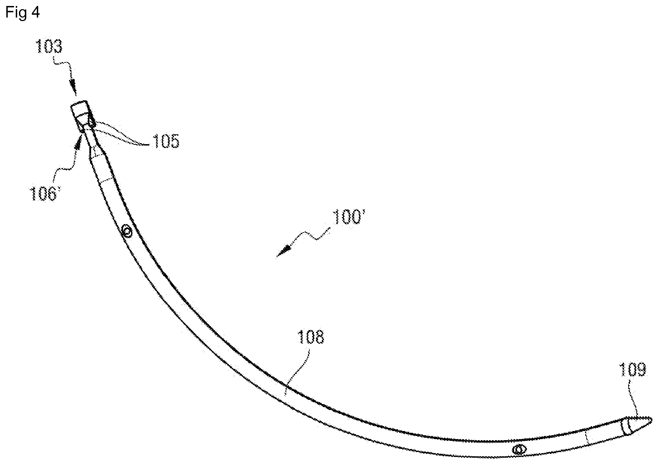

La figure 4 est une vue en perspective d'un autre mode de réalisation du bouchon, dans lequel le bouchon est de forme allongée et remplit en outre la fonction de guide d'insertion du tube 3 lorsque le bouchon allongé 100' et le tube 3 sont connectés lors de l'implantation.

Les signes de référence communs avec la figure 2 désignent des éléments similaires ou remplissant la même fonction et qui ne seront donc pas décrits à nouveau.

Selon cette réalisation particulière de l'invention, le bouchon 100' présente une portion de connexion similaire à celle du bouchon de la figure 2. Ladite portion de connexion comprend la gorge annulaire 103 et l'embout 102.

En revanche, du côté opposé à la portion de connexion selon la direction longitudinale, le bouchon 100' se prolonge par une tige de forme allongée 108, dont la longueur est typiquement de plusieurs centimètres. La tige 108 est pleine et présente avantageusement une extrémité 109 conique mais non pointue pour faciliter l'introduction dans les tissus sans les endommager de manière significative. Grâce à cette tige 108, le passage du tube 3 au travers des tissus à traverser entre les deux sites d'implantation, celui pour la manchette 1 et celui pour le boîtier 8, est facilité.

D'une manière générale, le bouchon et le collet sont dépourvus d'arêtes vives mais présentent des congés de raccordement afin de minimiser le risque d'endommager les tissus traversés.

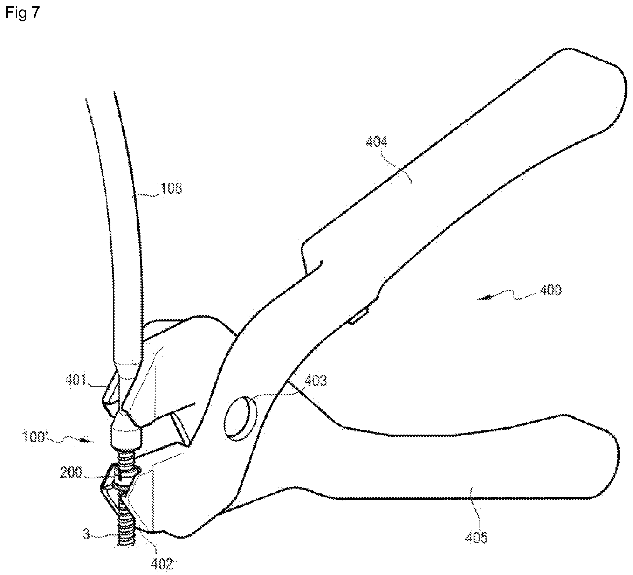

Comme le bouchon 100 de la figure 2, le bouchon 100' comprend des zones de préhension 105 qui permettent l'utilisation d'une pince pour la manipulation du bouchon ou pour emboîter le bouchon sur le collet (cf. figure 7).

Ladite pince comprend typiquement un premier mors adapté pour recevoir une portion du bouchon opposée à la portion de connexion dudit bouchon selon la direction longitudinale, un second mors adapté pour recevoir une portion du collet opposée à la portion de connexion dudit collet selon la direction longitudinale, lesdits mors étant mobiles en pivotement autour d'un axe d'articulation. Du côté opposé à chaque mors par rapport à l'axe d'articulation, la pince comprend deux poignées adaptées pour la préhension de la pince par un praticien. Dans la position ouverte de la pince, les mors sont écartés l'un de l'autre d'une distance suffisante pour permettre l'engagement du collet et du bouchon avec chaque mors respectif. Lors de la fermeture de la pince, les mors maintiennent le bouchon en regard du collet ; le praticien bénéficie d'un bras de levier pour permettre un emboîtement aisé du collet et du bouchon par leurs portions de connexion respectives.

De manière particulièrement avantageuse, l'accessoire comprend en outre un capuchon 300 adapté pour entourer les zones de préhension 105 de sorte à assurer une continuité de la surface extérieure du bouchon en regard desdites zones de préhension, après que le bouchon a été emboîté sur le collet, par exemple au moyen d'une pince. Cette continuité de la surface extérieure dans la direction longitudinale du bouchon permet d'éviter d'agresser les tissus.

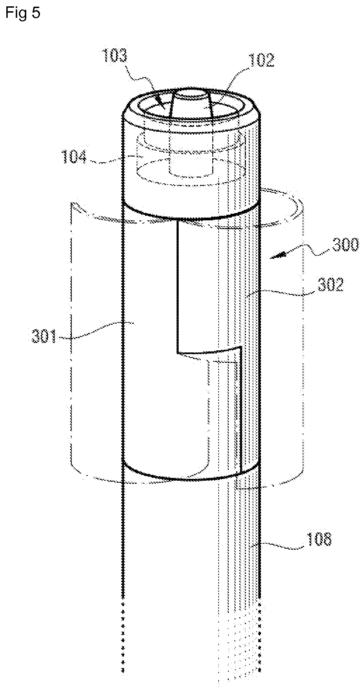

Selon un mode de réalisation illustré sur la figure 5, ledit capuchon 300 comprend deux demi-coques 301, 302 emboîtables de part et d'autre du bouchon. Lorsque lesdites demi-coques sont assemblées autour de la zone de préhension du bouchon, elles présentent une surface externe cylindrique sensiblement de même diamètre que la tige 108. De préférence, lesdites demi-coques 301, 302 sont solidaires l'une de l'autre et articulées par une charnière. Ainsi, le praticien a un seul objet à manipuler pour mettre en place les deux demi-coques autour de la zone de préhension du bouchon, ce qui améliore l'ergonomie.

La figure 6 illustre une étape de connexion du bouchon 100 de la figure 2 avec le collet 200 au moyen d'une pince 400.

La pince 400 comprend un premier mors 401 adapté pour recevoir une portion du bouchon 100 opposée à la portion de connexion dudit bouchon selon la direction longitudinale. De préférence, le premier mors comprend à cet effet un logement adapté pour recevoir les zones de préhension 105 du bouchon ou pour prendre appui sur la collerette 106.

La pince comprend un second mors 402 adapté pour recevoir une portion du collet 200 opposée à la portion de connexion dudit collet selon la direction longitudinale. Le second mors 402 comprend à cet effet un logement adapté pour recevoir la collerette 202 du collet. Ledit logement présente une ouverture traversante pour le passage du tube 3 sur lequel le collet 200 a été préalablement emmanché.

Lesdits mors 401, 402 sont mobiles en pivotement autour d'un axe d'articulation 403.

La pince 400 comprend en outre des poignées 405, 404 opposées respectivement aux mors 401, 402 par rapport à l'axe d'articulation 403.

Sur la figure 6, la pince est représentée en position ouverte, les mors étant écartés d'une distance suffisante pour permettre d'engager le bouchon et le collet dans lesdits mors.

Pour connecter le bouchon et le collet, l'utilisateur exerce une pression sur les poignées 404, 405 de sorte à les rapprocher, rapprochant ainsi les mors qui, grâce à la pression qu'ils exercent sur le collet et le bouchon, permettent l'engagement mutuel de leurs portions de connexion.

La figure 7 illustre une étape de connexion du bouchon 100' de la figure 4 avec le collet 200 au moyen de la pince 400, qui est identique à celle de la figure 6. La pince ne sera donc pas décrite en détail à nouveau.

Dans ce mode de réalisation, de préférence le bouchon 100' est maintenu dans le logement du premier mors 401 par ses zones de préhension 105 qui viennent en appui par leur bord 106' opposé à la portion de connexion contre une surface du mors. Ledit logement présente une ouverture traversante pour permettre le passage de la tige 108.

La pince peut être fournie au praticien avec un ou plusieurs bouchons et un ou plusieurs collets, sous la forme d'un kit d'implantation.

Il est entendu que les modes de réalisation décrits ci-dessus correspondent à des exemples particuliers et non-limitatifs et que diverses modifications peuvent être apportées sans pour autant s'éloigner de l'invention.

## Revendications

1. Accessoire pour une implantation d'un dispositif médical dans un corps humain ou animal, ledit dispositif médical comprenant une manchette occlusive (1) adaptée pour entourer et obturer un conduit anatomique dudit corps humain ou animal, un réservoir de fluide (2) et un tube (3) reliant le réservoir à la manchette occlusive, ledit accessoire comprenant :
- un bouchon (100 ; 100') adapté pour obturer une extrémité du tube, et
- un collet (200) adapté pour entourer le tube,
le bouchon (100) et le collet (200) présentant des portions de connexion respectives (103, 104 ; 203, 204) adaptées pour coopérer de manière à fixer le collet (200) et le bouchon (100) ensemble de sorte à obturer hermétiquement ladite extrémité du tube (3), et **caractérisé en ce que** le bouchon (100 ; 100') comprend un embout (102) adapté pour être inséré dans une extrémité du tube, et **en ce que** la portion de connexion du bouchon (100 ; 100') comprend une gorge annulaire (103) entourant ledit embout (102) configurée pour recevoir une extrémité déformable élastiquement du collet (200) de sorte à pincer l'extrémité du tube.

2. Accessoire selon la revendication 1, dans lequel les portions de connexion (103, 104 ; 203, 204) du bouchon et du collet sont agencées de sorte à permettre un emboîtement du collet avec le bouchon.

3. Accessoire selon l'une des revendications 1 ou 2, dans lequel l'une des portions de connexion du collet (200) ou du bouchon (100 ; 100') présente au moins une protrusion (204) et l'autre portion de connexion du collet ou du bouchon présente au moins une cavité (104), ladite au moins une protrusion étant déformable élastiquement pour permettre l'engagement de ladite au moins une protrusion dans ladite au moins une cavité.

4. Accessoire selon l'une des revendications 1 à 3, dans lequel le bouchon (100') comprend en outre une tige (108) allongée de sorte à former un guide d'insertion du tube lors de l'implantation.

5. Accessoire selon l'une des revendications 1 à 4, dans lequel le bouchon (100 ; 100') présente au moins une zone de préhension (105) pour une pince (300) configurée pour connecter le collet au bouchon.

6. Accessoire selon la revendication 5, dans lequel la zone de préhension (105) comprend deux méplats parallèles bordés par une collerette (106).

7. Accessoire selon la revendication 6, dans lequel lesdits méplats sont traversés par un orifice (107) de passage d'une suture.

8. Accessoire selon l'une des revendications 5 à 7, comprenant un capuchon (300) adapté pour entourer le bouchon (100') de sorte à assurer une continuité de la surface extérieure du bouchon en regard de la zone de préhension (105).

9. Accessoire selon la revendication 8, dans lequel ledit capuchon (300) comprend deux demi-coques (301, 302) emboîtables de part et d'autre du bouchon.

10. Accessoire selon la revendication 9, dans lequel lesdites demi-coques (301, 302) sont articulées par une charnière.

11. Kit d'implantation comprenant un accessoire selon l'une des revendications 1 à 10, et une pince (400) incluant un premier mors (401) adapté pour recevoir une portion du bouchon (100 ; 100') opposée à la portion de connexion dudit bouchon et un second mors (402) adapté pour recevoir une portion du collet (200) opposée à la portion de connexion dudit collet, lesdits mors étant mobiles entre une position ouverte adaptée pour l'engagement du collet et du bouchon avec chaque mors respectif et une position fermée adaptée pour connecter le collet et le bouchon par leurs portions de connexion respectives.

12. Ensemble comprenant :
- un dispositif médical adapté pour être implanté dans un corps humain ou animal, ledit dispositif médical comprenant une manchette occlusive (1) adaptée pour entourer et obturer un conduit anatomique dudit corps humain ou animal, un réservoir (2) de fluide et un tube (3) reliant le réservoir (2) à la manchette occlusive, et
- un accessoire selon l'une des revendications 1 à 10 pour l'implantation dudit dispositif médical dans un corps humain ou animal.

## Patentansprüche

1. Zubehör für eine Implantation einer medizinischen Vorrichtung in einen menschlichen oder tierischen Körper, wobei die medizinische Vorrichtung eine Verschlussmanschette (1) umfasst, die geeignet ist, einen anatomischen Kanal des menschlichen oder tierischen Körpers zu umgeben und zu verschließen, wobei ein Fluidbehälter (2) und ein Schlauch (3) den Behälter mit der Verschlussmanschette verbinden, wobei das Zubehör umfasst:
- einen Stopfen (100; 100'), der geeignet ist, ein Ende des Schlauchs zu verschließen, und
- einen Kragen (200), der geeignet ist, den Schlauch zu umgeben,
wobei der Stopfen (100) und der Kragen (200) jeweilige Verbindungsabschnitte (103, 104; 203, 204) aufweisen, die geeignet sind, derart zusammenzuwirken, dass der Kragen (200) und der Stopfen (100) gemeinsam derart verbunden werden, dass das Ende des Schlauchs (3) hermetisch verschlossen wird, und **dadurch gekennzeichnet, dass** der Stopfen (100; 100') ein Endstück (102) umfasst, das geeignet ist, in ein Ende des Schlauchs eingeführt zu werden, und dass der Verbindungsabschnitt des Stopfens (100; 100') eine ringförmige Nut (103) umfasst, die das Endstück (102) umgibt, die ausgelegt ist, um ein elastisch verformbares Ende des Kragens (200) derart aufzunehmen, dass das Ende des Schlauchs eingeklemmt wird.

2. Zubehör nach Anspruch 1, wobei die Verbindungsabschnitte (103, 104; 203, 204) des Stopfens und des Kragens so eingerichtet sind, dass ein Einrasten des Kragens mit dem Stopfen gestattet ist.

3. Zubehör nach einem der Ansprüche 1 oder 2, wobei einer der Verbindungsabschnitte des Kragens (200) oder des Stopfens (100; 100') mindestens einen Vorsprung (204) aufweist und der andere Verbindungsabschnitt des Kragens oder des Stopfens mindestens eine Vertiefung (104) aufweist, wobei der mindestens eine Vorsprung elastisch verformbar ist, um das Eingreifen des mindestens einen Vorsprungs in die mindestens eine Vertiefung zu gestatten.

4. Zubehör nach einem der Ansprüche 1 bis 3, wobei der Stopfen (100') ferner einen länglichen Schaft (108) umfasst, so dass beim Einsetzen eine Einführführung des Schlauchs gebildet wird.

5. Zubehör nach einem der Ansprüche 1 bis 4, wobei der Stopfen (100; 100') mindestens eine Greifzone (105) für eine Zange (300) aufweist, die ausgelegt ist, um den Kragen mit dem Stopfen zu verbinden.

6. Zubehör nach Anspruch 5, wobei der Greifbereich (105) zwei parallele Abflachungen umfasst, die von einem Hals (106) begrenzt werden.

7. Zubehör nach Anspruch 6, wobei die Abflachungen von einer Durchgangsöffnung (107) für eine Naht durchquert werden.

8. Zubehör nach einem der Ansprüche 5 bis 7, das eine Kappe (300) umfasst, die geeignet ist, den Stopfen (100') derart zu umgeben, dass eine Kontinuität der der Greifzone (105) zugewandten Außenfläche des Stopfens gewährleistet ist.

9. Zubehör nach Anspruch 8, wobei die Kappe (300) zwei Halbschalen (301, 302) umfasst, die auf beiden Seiten des Stopfens einrastbar sind.

10. Zubehör nach Anspruch 9, wobei die Halbschalen (301, 302) durch ein Scharnier angelenkt sind.

11. Implantationsset, umfassend ein Zubehör nach einem der Ansprüche 1 bis 10 und eine Zange (400) mit einer ersten Backe (401), die zur Aufnahme eines Abschnitts des Stopfens (100; 100') gegenüber dem Verbindungsabschnitt des Stopfens geeignet ist, und eine zweite Backe (402), die zur Aufnahme eines Abschnitts des Kragens (200) gegenüber dem Verbindungsabschnitt des Kragens geeignet ist, wobei die Backen zwischen einer offenen Position, die zum Eingreifen des Halses und des Stopfens mit jeder jeweiligen Backe geeignet ist, und einer geschlossenen Position, die zum Verbinden des Halses und des Stopfens mittels ihrer jeweiligen Verbindungsabschnitte geeignet ist, beweglich sind.

12. Anordnung, umfassend:
- eine medizinische Vorrichtung, die zum Implantieren in einen menschlichen oder tierischen Körper geeignet ist, wobei die medizinische Vorrichtung eine Verschlussmanschette (1), die geeignet ist, einen anatomischen Kanal des menschlichen oder tierischen Körpers zu umgeben und zu verschließen, einen Fluidbehälter (2) und einen Schlauch (3), der den Behälter (2) mit der Verschlussmanschette verbindet, umfasst, und
- ein Zubehör nach einem der Ansprüche 1 bis 10 zum Implantieren der medizinischen Vorrichtung in einen menschlichen oder tierischen Körper.

## Claims

1. An accessory for implanting a medical device in a human or animal body, said medical device comprising an occlusive cuff (1) adapted to surround and plug an anatomical duct of said human or animal body, a fluid reservoir (2) and a tube (3) connecting the reservoir to the occlusive cuff, said accessory comprising:
- a stopper (100; 100') adapted to plug one end of the tube, and
- a collar (200) adapted to surround the tube,
the stopper (100) and the collar (200) having respective connection portions (103, 104; 203, 204) adapted to cooperate to attach the collar (200) and the stopper (100) together so as to hermetically plug said end of the tube (3),
and **characterized in that** the stopper (100; 100') comprises an end piece (102) adapted to be inserted into an end of the tube, and **in that** the connection portion of the stopper (100; 100') comprises an annular groove (103) surrounding said end piece (102) configured to receive an elastically deformable end of the collar (200) so as to pinch the end of the tube.

2. The accessory according to claim 1, wherein the connection portions (103, 104; 203, 204) of the stopper and the collar are arranged so as to allow interlocking of the collar with the stopper.

3. The accessory according to one of claims 1 or 2, wherein one of the connection portions of the collar (200) or the stopper (100; 100') has at least one protrusion (204) and the other connection portion of the collar or the stopper has at least one cavity (104), said at least one protrusion being elastically deformable to allow engagement of said at least one protrusion in said at least one cavity.

4. The accessory according to one of claims 1 to 3, wherein the stopper (100') further comprises an elongated rod (108) so as to form a guide for insertion of the tube upon implantation.

5. The accessory according to one of claims 1 to 4, wherein the stopper (100; 100') has at least one gripping zone (105) for pliers (300) configured to connect the collar to the stopper.

6. The accessory according to claim 5, wherein the gripping zone (105) comprises two parallel flats bordered by a flange (106).

7. The accessory according to claim 6, wherein said flats have a suture passage hole (107) therethrough.

8. The accessory according to one of claims 5 to 9, comprising a cap (300) adapted to surround the stopper (100') so as to ensure continuity of the outer surface of the stopper facing the gripping zone (105).

9. The accessory according to claim 8, wherein said cap (300) comprises two interlocking half-shells (301, 302) on either side of the stopper.

10. The accessory according to claim 9, wherein said half-shells (301, 302) are hinged by a hinge.

11. An implantation kit comprising an accessory according to one of claims 1 to 10, and pliers (400) including a first jaw (401) adapted to receive a portion of the stopper (100; 100') opposite to the connection portion of said stopper and a second jaw (402) adapted to receive a portion of the collar (200) opposite to the connection portion of said collar, said jaws being movable between an open position adapted for engagement of the collar and the stopper with each respective jaw and a closed position adapted to connect the collar and the stopper through their respective connection portions.

12. An assembly comprising:
- a medical device adapted to be implanted in a human or animal body, said medical device comprising an occlusive cuff (1) adapted to surround and plug an anatomical duct of said human or animal body, a fluid reservoir (2) and a tube (3) connecting the reservoir (2) to the occlusive cuff, and
- an accessory according to one of claims 1 to 10 for implanting said medical device in a human or animal body.
